# FASCICULE DE BREVET EUROPEEN

(11) **EP 1 032 558 B1**
(45) Date de publication et mention de la délivrance du brevet: **25.02.2004**
(21) Numéro de dépôt: 98955702.0
(22) Date de dépôt: 19.11.1998
(51) Int. Cl.: C07C 253/30, B01J 10/00, B01J 19/24, C07C 209/48

(54) **PROCEDE D'HYDROGENATION DE DINITRILES**
VERFAHREN ZUR HYDRIERUNG VON DINITRILEN
METHOD FOR HYDROGENATING DINITRILES

(30) Priorité: 20.11.1997 FR 9714809
(43) Date de publication de la demande: 06.09.2000
(73) Titulaire: Rhodia Polyamide Intermediates, 69192 Saint-Fons (FR)
(72) Inventeur: BOSCHAT, Vincent, F-69007 Lyon (FR); LECONTE, Philippe, F-69330 Meyzieu (FR); ROCHETTE, Daniel, F-38550 Saint-Maurice l'Exil (FR); SEVER, Lionel, F-38200 Luzinay (FR)
(74) Mandataire: Esson, Jean-Pierre
(86) Numéro de dépôt international: PCT/FR1998/002479
(87) Numéro de publication internationale: WO 1999/026917

(56) Documents cités:
- EP-A- 0 070 797
- WO-A-93/12073
- WO-A-93/16034
- WO-A-96/18603
- WO-A-98/11060
- US-A- 5 151 543
- US-A- 5 508 465
- US-A- 5 675 045

## Description

La présente invention concerne un procédé d'hydrogénation de dinitriles aliphatiques au moins en partie en aminonitriles correspondants.

L'hydrogénation des dinitriles en diamines correspondantes est une technique connue depuis longtemps, notamment l'hydrogénation de l'adiponitrile en hexaméthylènediamine, l'une des matières de base de la préparation du polyamide 66.

Depuis quelques années est apparu un intérêt croissant à l'hydrogénation (aussi parfois appelée hémihydrogénation) des dinitriles aliphatiques en aminonitriles, notamment l'hydrogénation de l'adiponitrile en 6-aminocapronitrile conduisant soit directement, soit par l'intermédiaire du caprolactame, au polyamide 6.

Ainsi le brevet US-A-5 151 543 décrit un procédé d'hydrogénation sélective de dinitriles aliphatiques en aminonitriles correspondants, à 25-150°C et sous pression supérieure à la pression atmosphérique, en présence d'un solvant en excès molaire d'au moins 2/1 par rapport au dinitrile, le solvant contenant de l'ammoniac liquide ou un alcool de 1 à 4 atomes de carbone contenant une base minérale soluble dans ledit alcool et en présence d'un catalyseur Raney, l'aminonitrile obtenu étant récupéré comme produit principal.

Le brevet WO-A-93/16034 décrit un procédé de préparation de 6-aminocapronitrile par hydrogénation de l'adiponitrile, en présence d'une base minérale, d'un complexe de métal de transition de faible valence choisi parmi le chrome, le tungstène, le cobalt et le fer et de nickel de Raney comme catalyseur, sous pression d'hydrogène et à une température de 50°C à 90°C.

Le brevet WO-A-96/18603 décrit l'hémihydrogénation de dinitriles aliphatiques en aminonitriles, par l'hydrogène et en présence d'un catalyseur à base de nickel ou de cobalt de Raney éventuellement dopé et d'une base minérale forte, le milieu initial d'hydrogénation comportant de l'eau, de l'aminonitrile et/ou de la diamine susceptibles de se former et du dinitrile non transformé.

Tous ces procédés d'hydrogénation conduisent à l'aminonitrile recherché et sont présentés comme pouvant être mis en oeuvre en continu dans une installation industrielle.

Cependant certains problèmes n'ont pas été mis en évidence pour une application industrielle. Ainsi, au cours des recherches effectuées dans ce domaine par la Demanderesse, il a été constaté que les catalyseurs d'hydrogénation, et en particulier le nickel de Raney, le cobalt de Raney, les métaux supportés, notamment les métaux du groupe VIII de la classification périodique des éléments tels que le nickel, le cobalt, le ruthénium, le rhodium, déposés sur un support qui est généralement un oxyde. présentent une tendance marquée à se désactiver plus rapidement lorsqu'ils se trouvent en présence de fonctions nitrile et en l'absence d'hydrogène.

Ce problème ne se pose pratiquement pas dans les procédés industriels actuels qui conduisent à la diamine, tandis que l'hydrogénation en aminonitrile conduit à conserver en permanence dans le milieu réactionnel une quantité importante de fonctions nitrile. Dans un tel procédé, il faut récupérer les produits de la réaction, aminonitrile et diamine, ainsi que le dinitrile non transformé, tout en maintenant ou en recyclant la majeure partie du catalyseur tant qu'il est suffisamment actif. Il faut donc, compte-tenu des observations précédentes, à la fois séparer l'aminonitrile et la diamine formés et le dinitrile non transformé pour les récupérer, et maintenir ou recycler le catalyseur sans occasionner une désactivation supplémentaire. Cela implique donc d'avoir des conditions opératoires et un appareillage permettant une séparation relativement rapide, compatible avec une exploitation industrielle, du catalyseur et de la partie liquide du mélange réactionnel et également que ladite séparation n'entraîne pas une désactivation trop importante dudit catalyseur.

On pourrait envisager une séparation d'une partie du mélange réactionnel contenant le catalyseur, par filtration ou centrifugation, mais selon les observations de la Demanderesse le catalyseur ainsi manipulé en absence d'hydrogène et en présence de fonctions nitrile perd partiellement son activité ; la diminution de la durée de vie du catalyseur pèse alors défavorablement sur l'économie du procédé. Par contre, la filtration sous pression d'hydrogène, donc en présence d'hydrogène dissous, permet d'éviter la désactivation du catalyseur.

La présente invention propose une solution à ces différents problèmes. Elle consiste plus précisément en un procédé continu d'hydrogénation de dinitrile, au moins en partie en aminonitrile correspondant, en présence d'un catalyseur d'hydrogénation non-dissous dans le milieu réactionnel, caractérisé en ce qu'il consiste en sortie du réacteur d'hydrogénation de réaliser une séparation du gaz contenu dans le milieu réactionnel, dans une zone où le transfert gaz-liquide est limité ou nul, à reconduire le milieu réactionnel appauvrie en gaz dans ledit réacteur d'hydrogénation et à soutirer une partie dudit milieu réactionnel de ladite zone de séparation liquide-gaz après séparation du solide dans une zone de séparation liquide:solide, avec recyclage du solide décanté dans le réacteur d'hydrogénation avec un temps de séjour inférieur à 30 minutes du solide décanté dans ladite zone de séparation liquide-solide.

L'appareillage convenant à la mise en oeuvre de la séparation du gaz dumilieu réactionnel du procédé de l'invention permet de réaliser un excellent contact gaz/liquide, une séparation rapide et efficace de ces deux phases après contact, une séparation continue de l'hydrogénat et du catalyseur et le recyclage de ce dernier, en un temps compatible avec une déactivation la plus faible possible dudit catalyseur.

Ledit appareillage comporte trois sections principales: une section réaction, une section séparation gaz-liquide et une section séparation catalyseur-liquide avec recyclage dudit catalyseur et soutirage du liquide (hydrogénat).

La section réaction comporte généralement un ou plusieurs tubes en forme de U dont les branches sont verticales ou légèrement inclinées par rapport à la verticale, l'une des branches de chaque U assurant la montée de la dispersion gaz/liquide/catalyseur solide, l'autre le retour du liquide au moins partiellement dégazé. Elle comporte également quatre arrivées à la base de la branche montante : l'arrivée d'hydrogène, l'arrivée du dinitrile, l'arrivée du catalyseur, neuf ou régénéré, avec ou sans cocatalyseur, l'arrivée du catalyseur recyclé.

La section séparation gaz-liquide est constituée d'un cylindre vertical comportant une ou plusieurs arrivées tangentielles (venant de la branche montante du réacteur), un ou plusieurs départs tangentiels (vers la branche descendante du réacteur), une sortie de gaz et une sortie du mélange réactionnel vers la séparation liquide-solide. L'arrivée de la dispersion gaz/liquide/catalyseur solide s'effectue en un point situé au-dessus de point de départ du liquide dégazé.

La section séparation liquide-solide est constituée d'un décanteur et/ou d'un filtre ce qui permet de séparer l'hydrogénat du catalyseur et de recycler ledit catalyseur. L'hydrogénat est soutiré en continu tandis que la suspension de catalyseur séparée dans le décanteur et/ou dans le filtre est ramenée dans la section réaction. Une purge est réalisée lorsqu'il est jugé nécessaire de remplacer une partie du catalyseur par du catalyseur neuf.

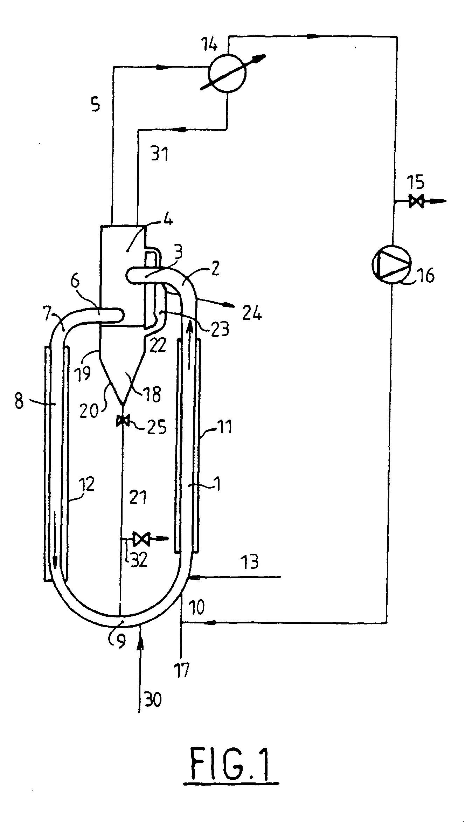

L'appareillage convenant au procédé de l'invention peut être illustré à titre d'exemple par la figure 1. Il comporte un tube vertical cylindrique (1) relié par un tube coudé (2) à un tube horizontal (3) débouchant tangentiellement dans un séparateur gaz/liquide (4) constitué par un cylindre vertical de diamètre supérieur à celui du tube (1).

Le séparateur (4) comporte une tubulure (5) d'évacuation de gaz ou de vapeurs. Un tube horizontal (6) prenant naissance tangentiellement au séparateur et en un point situé au-dessous du point d'arrivée du tube (3), est relié par l'intermédiaire d'un tube coudé (7) à un second tube vertical (8) communiquant avec le tube (1) par un coude (9). L'ensemble des tubes (1) et (8) et du coude (9) forme un U. Le tube (1) comporte à sa base une tubulure (10) d'introduction d'hydrogène et une tubulure (13) d'introduction du dinitrile. Les tubes (1) et (8) peuvent comporter, comme représenté sur la figure 1, une double enveloppe (11) et (12) permettant la circulation d'un fluide de refroidissement ou de réchauffage. Le coude (9) comporte une arrivée (30) de catalyseur neuf ou régénéré et une arrivée (21) de catalyseur recyclé.

Les tubes (1) et (8) peuvent être verticaux ou légèrement obliques (dans ce dernier cas de préférence de telle sorte que leurs axes convergent vers le bas).

Les rayons de courbure des coudes 2, 7, 9 sont calculés selon les règles habituelles du génie chimique, de manière que la perte de charge de la masse en circulation dans l'ensemble du circuit soit aussi faible que possible. Leur angle de courbure peut varier de 45° à 135° et de préférence de 60° à 120°.

Sur la figure 1 l'hydrogène est introduit par une tubulure (10). Cette tubulure peut être munie de tout dispositif usuel de dispersion, mais un simple tube affleurant la paroi, disposé dans l'axe du tube (1) est suffisant. Cette tubulure (10) est reliée à une source d'hydrogène et celui-ci peut être introduit à la pression atmosphérique ou sous une pression supérieure.

La tubulure (5) d'évacuation des gaz peut être reliée à un dispositif quelconque de traitement des gaz séparés de l'hydrogénat. La figure 1 illustre un dispositif selon lequel les gaz issus de (5) passent dans un condenseur (14), dans lequel les vapeurs entraînées dans le séparateur (4) sont séparées de l'hydrogène. Le condensat obtenu est recyclé dans l'appareil par une tubulure (31). L'hydrogène en excès passe ensuite dans un compresseur (16) par une canalisation comportant un système de purge (15), puis il est recyclé en (10) après introduction en (17) d'une quantité d'hydrogène destinée à compenser l'hydrogène consommé au cours de l'hydrogénation et celui qui a été purgé.

Il est nécessaire de soutirer l'hydrogénat formé dégazé et débarrassé du catalyseur Pour pouvoir soutirer un hydrogénat clair, c'est-à-dire ne comportant pratiquement pas de catalyseur, un décanteur (18) est placé directement sous le séparateur (4). La suspension liquide/catalyseur dont la phase gazeuse a été séparée dans le séparateur (4) pénètre dans le décanteur (18).

Le décanteur (18) est constitué par un cylindre (19) terminé par un cône (20). Une canalisation (21) sert à ramener en continu dans le coude (9) la bouillie concentrée de catalyseur. L'hydrogénat débarrassé du catalyseur sort par une canalisation (22) reliée à un pôt (23) muni d'un trop-plein (24) permettant de soutirer en continu l'hydrogénat clair, le niveau dans l'ensemble de l'appareil étant maintenu constant par l'introduction continue d'un volume équivalent de mélange dinitrile, solvant et catalyseur. Le débit dans le tube (21) est réglé au moyen d'une vanne (25), de manière que la bouillie liquide/catalyseur conserve une concentration adéquate. La tubulure (21) comporte une tubulure (32) de purge du catalyseur usé, qui pourra éventuellement être régénéré.

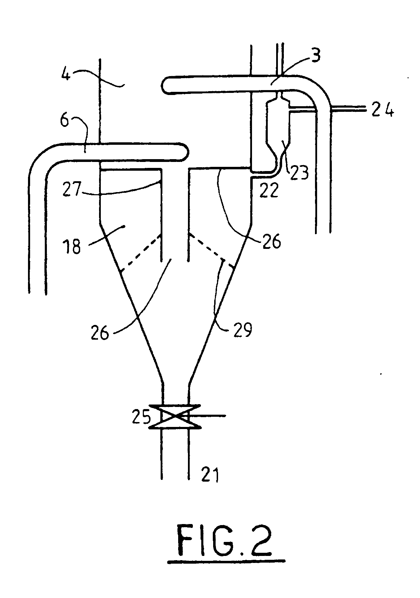

La figure 2 illustre un mode particulier de décantation entrant dans le cadre de l'invention. Pour éviter, d'une part, que les mouvements trop rapides de la masse de catalyseur et d'hydrogénat ne se propagent dans le décanteur (18) et, d'autre part, que l'hydrogène ne pénètre dans ce dernier, une séparation entre les deux zones (section séparation gaz-liquide et section séparation liquide-solide) est nécessaire. Elle ne doit cependant en aucune façon être la cause d'un dépôt de catalyseur. Un tel résultat est obtenu grâce à la mise en place entre le séparateur (4) et le décanteur (18) d'une cloison (26), la circulation entre le séparateur gaz-liquide et le décanteur étant assurée par une canalisation (27) de diamètre calculé pour réduire notablement la vitesse du liquide (par exemple à une valeur inférieure à 0,5 mètre/seconde). Cette canalisation (27) se prolonge à l'intérieur du décanteur par un tube (28) de diamètre supérieur ou égal à celui de la canalisation (27). Une toile métallique (29) à larges mailles, en forme de cône dirigé vers le haut peut être placée à l'intérieur du décanteur (18) pour atténuer les turbulences créées par l'arrivée de la bouillie liquide/catalyseur.

L'appareillage décrit précédemment peut être modifié en ajoutant un filtre au décanteur ou en remplaçant ledit décanteur par un filtre.

La mise en oeuvre de l'appareillage décrit précédemment au procédé d'hémihydrogénation de l'adiponitrile en 6-aminocapronitrile permet d'obtenir une bonne dispersion de l'hydrogène dans le mélange liquide réactionnel. Cette dispersion est stable et homogène dans tout le tube ou les tubes en U. Cet appareillage permet comme cela a été dit précédemment de séparer en continu et sans désactivation importante du catalyseur l'hydrogénat à soutirer dudit catalyseur à recycler. En effet le temps de séjour du catalyseur dans le décanteur (18) et de recyclage dudit catalyseur peut être limité en moyenne à une valeur inférieure ou égale à 30 minutes, de préférence inférieure ou égale à 15 minutes et encore plus préférentiellement inférieure ou égale à 5 minutes. Dans le cas où le décanteur est remplacé par un filtre ou est complété par l'adjonction d'un filtre, ces durées de séjour sont également respectées.

Afin d'obtenir une bonne décantation du catalyseur dans les limites de durée indiquées précédemment, on mettra en oeuvre de préférence une concentration moyenne en catalyseur, correspondant au rapport pondéral catalyseur/mélange réactionnel liquide, supérieure à 5 %. Pour des concentrations plus faibles, on tend à entraîner une partie du catalyseur avec l'hydrogénat soutiré, lorsque l'on respecte la durée maximale de séjour dans le décanteur (18). Dans ce cas, la technique de filtration peut s'avérer avantageuse.

Une concentration moyenne en catalyseur égale ou supérieure à 10 % est encore préférée, car elle permet une décantation du catalyseur encore plus rapide, donc un maintien dudit catalyseur dans des conditions défavorables pendant un temps plus court, par exemple inférieur ou égal à 15 minutes.

Les dinitriles aliphatiques qui peuvent être mis en oeuvre dans le procédé de l'invention sont plus particulièrement les dinitriles de formule générale (I) :

NC-R-CN (I)

dans laquelle R représente un groupement alkylène ou alcénylène, linéaire ou ramifié, ayant de 1 à 12 atomes de carbone.

De préférence, on met en oeuvre dans le procédé de l'invention des dinitriles de formule (I) dans laquelle R représente un radical alkylène, linéaire ou ramifié ayant de 1 à 6 atomes de carbone.

A titre d'exemples de tels dinitriles, on peut citer notamment l'adiponitrile, le méthylglutaronitrile, l'éthylsuccinonitrile, le malononitrile, le succinonitrile et le glutaronitrile et leurs mélanges, notamment les mélanges d'adiponitrile et/ou de méthylglutaronitrile et/ou d'éthylsuccinonitrile susceptibles de provenir d'un même procédé de synthèse de l'adiponitrile.

En pratique, le cas où R = (CH₂)₄ sera le plus fréquent car cela correspond à la mise en oeuvre de l'adiponitrile (ADN) dans le présent procédé.

Le catalyseur est constitué en général d'un catalyseur à base de nickel de Raney et/ou de cobalt de Raney, comportant éventuellement, mais préférentiellement un ou plusieurs éléments dopants choisis parmi les éléments des groupes Ib, IVb, Vlb, VIIb et VIII de la classification périodique des éléments telle que publiée dans Handbook of Chemistry and Physics, 51st Edition (1970-1971).

Le catalyseur à base de nickel de Raney et/ou de cobalt de Raney utilisé dans le procédé peut donc comporter, outre le nickel ou le cobalt et les éventuelles quantités résiduelles du métal éliminé de l'alliage d'origine lorsque le catalyseur provient de l'attaque d'un alliage, c'est-à-dire généralement l'aluminium, un ou plusieurs autres éléments dopants, tels que par exemple le chrome, le titane, le molybdène, le tungstène, le fer, le zinc, le cuivre.

Parmi ces éléments dopants le chrome et/ou le fer et/ou le titane sont considérés comme les plus avantageux. Ces dopants représentent habituellement, en poids par poids de nickel ou de cobalt, de 0 % à 15 % et de préférence de 0 % à 10 %.

Le catalyseur peut aussi être constitué par un métal, qui est généralement un métal du groupe VIII de la classification périodique des éléments tel que le ruthénium, le rhodium, le nickel ou le cobalt, déposé sur un support qui est généralement un oxyde tel que les alumines, les silices, les aluminosilicates, le dioxyde de titane, l'oxyde de zirconium, l'oxyde de magnésium.

Dans les catalyseurs métalliques supportés, le métal représente généralement de 0,1 à 80 % du poids du support et préférentiellement de 0,5 à 50 %.

Le nickel de Raney, notamment le nickel de Raney dopé, est préféré dans le cadre de la présente invention.

Pour la composition du milieu réactionnel, notamment pour la nature et la quantité de solvant et de base, on pourra se référer plus particulièrement à ce qui est décrit dans le brevet WO-A-96/18603, dont le contenu est incorporé par référence dans la présente description ou encore dans le brevet EP-A-0 641 315.

Les exemples qui suivent illustrent l'invention.

### ESSAIS A ET B

Ces essais sont destinés à mettre en évidence la désactivation du Ni de Raney en présence de fonctions nitrile et en l'absence d'hydrogène.

Afin que la consommation "normale" du Ni, due à son action catalytique, n'interfère pas, seule la vitesse initiale de consommation de l'hydrogène lors de l'hydrogénation de l'adiponitrile (AdN) sera prise en compte.

Dans un réacteur, on charge 66,5 g d'AdN, 22,3 g d'HMD, 10 g d'eau, de la potasse à raison de 0,4 mol/kg de Ni et 1,2 g de Ni de Raney contenant 1,8 % en poids de Cr.

Dans l'essai A servant de témoin, l'hydrogénation est réalisée immédiatement à 50°C et sous 20 bar de pression d'hydrogène.

Dans l'essai B, l'hydrogénation est réalisée à 50°C et sous 20 bar de pression d'hydrogène, mais avant l'introduction de l'hydrogène, le mélange réactionnel est agité pendant 30 min sous azote à 50°C.

Les vitesses initiales d'hydrogénation mesurées pour ces deux essais sont les suivantes :
- essai A : 24 litres d'hydrogène consommés par heure
- essai B : 21 litres d'hydrogène consommés par heure.

Le maintien du catalyseur pendant 30 min en présence de fonctions nitrile et en l'absence d'hydrogène se traduit donc par une diminution de 12,5 % de l'activité initiale dudit catalyseur.

### ESSAI C

Afin de différencier dans les exemples 1 à 3 qui suivent la part de la consommation "normale" de Ni de Raney de celle éventuellement due à la désactivation dudit Ni de Raney en absence d'hydrogène, un essai continu d'hydrogénation de l'AdN a été réalisé.

L'essai consiste à charger dans un réacteur agité par une turbine, 143,6 g d'AdN, 201.1 g d'aminacapronitrile (ACN), 134,1 g d'hexaméthylènediamine (HMD), de la potasse à raison de 0,46 mol/kg de Ni et 6,4 g de Ni de Raney contenant 1,8 % en poids de Cr.

Après avoir purgé l'appareil à l'hydrogène, la température est réglée à 50°C et la pression d'hydrogène à 25 bar. Lorsque le mélange réactionnel est en température, on injecte de l'AdN avec un débit de 172,5 g/h et une solution aqueuse de potasse à 0,02 mol/l avec un débit de 16,4 g/h.

Le mélange réactionnel est soutiré en continu par passage au travers d'un filtre placé dans le réacteur. Le nickel n'est pas renouvelé, mais la séparation est réalisée dans cet essai dans des conditions de transfert gaz/liquide assurant au catalyseur une concentration d'hydrogène constante.

Après hydrogénation de 1216 g d'AdN, l'essai est arrêté et l'activité du Ni de Raney contenu dans le réacteur est mesurée à l'aide du test d'hydrogénation suivant.

On prélève 1 à 2 g de bouillie de Ni de Raney, on lave le catalyseur avec six fois 50 ml d'eau distillée, on pèse exactement 0,40 g de catalyseur dans un picnomètre. Ledit catalyseur est introduit dans un autoclave de 150 ml en acier inoxydable, muni d'un système d'agitation, d'un système de chauffage, de moyens d'introduction d'hydrogène et de réactifs et de moyens de mesure de la température et de la pression. Avec le catalyseur on entraîne aussi environ 0,4 g d'eau (cette quantité est prise en compte dans la composition pondérale des 42 g de solvant réactionnel composé de 90 % d'HMD et de 10 % d'eau). On charge dans l'autoclave, sous atmosphère d'argon, l'HMD, l'eau et de la potasse (à raison de 0,05 % en poids du mélange réactionnel). L'autoclave est purgé à l'azote et à l'hydrogène. Il est ensuite chauffé et est maintenu sous 25 bar d'hydrogène (par l'intermédiaire d'une réserve d'hydrogène). On met en route le système d'enregistrement de la pression d'hydrogène dans ladite réserve et on injecte rapidement 6 g d'AdN. L'hydrogénation est suivie jusqu'à la fin de consommation d'hydrogène.

Le test précédent est conduit d'une part avec du Ni de Raney neuf tel que mis en oeuvre dans l'essai C et d'autre part avec le Ni de Raney ayant servi à réaliser l'essai C (Ni usé). La vitesse initiale d'hydrogénation mesurée dans ce test représente l'activité du Ni de Raney. L'activité du Ni usé représente 40 % de l'activité du Ni neuf.

La consommation chimique de Ni correspondant à sa perte d'activité "normale" due à son utilisation comme catalyseur d'hydrogénation est calculée par le produit de la quantité de Ni chargée (6,4 g) par la différence d'activité avant et après l'essai C (1 - 0,4), le résultat étant divisé par la quantité d'AdN hydrogéné dans ledit essai C (1216 g) et est exprimée en kg de Ni/t d'AdN transformé.

Cette consommation chimique "normale" est de 3,15 kg Ni/t AdN.

### EXEMPLES 1 A 3

Les essais sont réalisés dans un appareillage tel que décrit dans les figures 1 et 2.

L'hydrogénation est réalisée sur l'adiponitrile, le solvant étant constitué par l'eau (8 % en poids par rapport à l'adiponitrile engagé). Le catalyseur est un nickel de Raney comportant 1,8 % en poids de dopant chrome par rapport au poids de Ni. On engage 15 % de catalyseur par rapport au mélange réactionnel.

Les flux d'alimentation de l'adiponitrile, du nickel de Raney neuf destiné à compenser la désactivation "normale" (usure) du catalyseur et de la solution de potasse (solution aqueuse à 50 % en poids) sont indiqués dans le tableau 1 ci-après.

La température (T°C) à laquelle est réalisé chaque essai, le taux de transformation de l'adiponitrile (AdN TT %), la sélectivité en amino-6 capronitrile par rapport à l'AdN transformé (ACN RT %), la sélectivité en hexaméthylènediamine par rapport à l'AdN transformé (HMD RT %), la consommation chimique de Ni (quantité de Ni entièrement désactivé qui a été remplacée : Cs Ni kg/t AdN transformé), l'activité résiduelle du Ni purgé (Act Ni : en % de l'activité initiale) sont rassemblés dans le tableau 1 ci-après.

Pour chacun des exemples, le temps moyen de séjour du catalyseur dans le décanteur (18) a été de 5 minutes.

La circulation d'hydrogène est de 9000 Nm³/h et la pression d'hydrogène est de 25 bar.

**Tableau 1**

| ALIMENTATION | | | REACTEUR | | | | | |
|---|---|---|---|---|---|---|---|---|
| AdN t/h | Ni neuf kg/h | KOH à 50 % kg/h | T°C | AdN TT% | ACN RT % | HMD RT % | Cs Ni kg/t AdN transformé | Act Ni % |
| 3,66 | 11 | 2,2 | 59 | 88,9 | 25,8 | 73 | 3,1 | 9 |
| 5,0 | 14 | 2,8 | 50 | 70,8 | 42,7 | 55,3 | 3,4 | 14 |
| 6,2 | 15 | 2,1 | 48 | 69,0 | 58,2 | 39,9 | 3,2 | 9 |

Les résultats obtenus avec le procédé de l'invention montrent que la consommation chimique de Ni de Raney n'est pas plus élevée que dans l'essai C réalisé en maintenant constamment le Ni en présence d'hydrogène. Le procédé de l'invention permet donc de réaliser en continu l'hydrogénation de dinitrile en aminonitrile sans désactivation du catalyseur autre que l'usure normale dudit catalyseur.

## Revendications

1. Procédé d'hydrogénation en continu de dinitrile pour produire au moins un aminonitrile correspondant, en présence d'un catalyseur d'hydrogénation non-dissous dans le milieu réactionnel **caractérisé en ce qu'**il consiste en sortie du réacteur d'hydrogénation de réaliser une séparation du gaz contenu dans le milieu réactionnel, à reconduire le milieu réactionnel appauvrie en gaz dans ledit réacteur d'hydrogénation et à soutirer l'hydrogénat de ladite zone de séparation liquide-gaz après séparation du catalyseur dans une zone de séparation liquide-solide, avec recyclage du catalyseur décanté dans le réacteur d'hydrogénation, ladite séparation gaz-liquide-solide étant réalisée dans une zone où le transfert gaz-liquide est limité ou nul et avec un temps de séjour inférieur à 30 minutes du catalyseur décanté dans ladite zone de séparation gaz-liquide-solide.

2. Procédé selon la revendication 1, **caractérisé en ce que** la séparation gaz-liquide est mise en oeuvre dans un appareillage formé par un cylindre vertical comprenant au moins une alimentation depuis le réacteur d'hydrogénation du milieu réactionnel d'hydrogénation disposée de manière tangentielle sur le cylindre vertical et au moins un recyclage du milieu réactionnel dégazé dans le réacteur d'hydrogénation disposé de matière tangentielle sur le cyclindre vertical à une position inférieure à celle de ladite arrivée.

3. Procédé selon la revendication 1 ou 2, **caractérisé en ce que** la séparation liquide-solide est réalisée par décantation ou filtration.

4. Procédé selon la revendication 3, **caractérisé en ce que** la suspension de catalyseur récupéré après décantation ou filtration est recyclé dans la zone de réaction.

5. Procédé selon l'une des revendications 1 à 4, **caractérisé en ce qu'**une partie du catalyseur recyclé à partir de la zone de séparation solide-liquide dans le réacteur d'hydrogénation est remplacé par du catalyseur neuf.

6. Procédé selon l'une des revendications précédentes, **caractérisé en ce que** le temps de séjour du catalyseur décanté dans la zone de séparation liquide-solide est inférieur à 15 minutes.

7. Procédé selon la revendication 6, **caractérisé en ce que** ledit temps de séjour est inférieur à 5 minutes.

8. Procédé selon l'une des revendications précédentes, **caractérisé en ce que** le gaz séparé de la zone de séparation liquide-gaz est recyclé dans le réacteur d'hydrogénation.

9. Procédé selon l'une des revendications précédentes, **caractérisé en ce que** le réacteur d'hydrogénation comprend un ou plusieurs tubes en forme de U dont les branches sont verticales ou légèrement inclinées par rapport à la verticale, l'une des branches de chaque U assurant la montée de la dispersion gaz/liquide/catalyseur solide vers la zone de séparation liquide-gaz, l'autre branche de chaque U recevant le milieu liquide au moins partiellement dégazé sortant de la zone de séparation liquide-gaz.

10. Procédé selon l'une des revendications 1 à 9, **caractérisé en ce que** la concentration moyenne en catalyseur, correspondant au rapport pondéral catalyseur/mélange réactionnel liquide, est supérieure à 5 % et de préférence égale ou supérieure à 10 %.

11. Procédé selon l'une des revendications 1 à 10, **caractérisé en ce que** le dinitrile aliphatique mis en oeuvre est choisi parmi les dinitriles de formule générale (I) :
NC―R―CN (I)
dans laquelle R représente un groupement alkylène ou alcénylène, linéaire ou ramifié, ayant de 1 à 12 atomes de carbone.

12. Procédé selon l'une des revendications 1 à 11, **caractérisé en ce que** le dinitrile aliphatique mis en oeuvre est choisi parmi l'adiponitrile, le méthylglutaronitrile, l'éthylsuccinonitrile, le malononitrile, le succinonitrile et le glutaronitrile et leurs mélanges, notamment les mélanges d'adiponitrile et/ou de méthylglutaronitrile et/ou d'éthylsuccinonitrile susceptibles de provenir d'un même procédé de synthèse de l'adiponitrile.

13. Procédé selon l'une des revendications 1 à 12 **caractérisé en ce que** le dinitrile aliphatique mis en oeuvre est l'adiponitrile.

14. Procédé selon l'une des revendications 1 à 13 **caractérisé en ce que** le catalyseur utilisé est constitué d'un catalyseur à base de nickel de Raney et/ou de cobalt de Raney, comportant éventuellement, mais préférentiellement un élément dopant choisi parmi les éléments des groupes Ib, IVb, VIb, VIIb et VIII de la classification périodique des éléments telle que publiée dans Handbook of Chemistry and Physics, 51st Edition (1970-1971).

15. Procédé selon la revendication 14, **caractérisé en ce que** le catalyseur à base de nickel de Raney et/ou de cobalt de Raney comporte, outre le nickel ou le cobalt, un ou plusieurs autres éléments dopants, choisis parmi le chrome, le titane, le molybdène, le tungstène, le fer, le zinc, le cuivre.

16. Procédé selon l'une des revendications 1 à 13 **caractérisé en ce que** le catalyseur utilisé est constitué par un métal du groupe VIII de la classification périodique des éléments tel que le ruthénium, le rhodium, le nickel ou le cobalt, déposé sur un support qui est un oxyde tel que les alumines, les silices, les aluminosilicates, le dioxyde de titane, l'oxyde de zirconium, l'oxyde de magnésium.

## Patentansprüche

1. Verfahren zur kontinuierlichen Hydrierung von Dinitril für die Herstellung von mindestens einem entsprechenden Aminonitril, in Anwesenheit eines in dem Reaktionsmedium nicht gelösten Hydrierungskatalysators, **dadurch gekennzeichnet, daß** es darin besteht, am Ausgang des Hydrierungsreaktors eine Abtrennung des in dem Reaktionsmedium enthaltenen Gases vorzunehmen, das an Gas abgereicherte Reaktionsmedium wieder in den genannten Hydrierungsreaktor zurückzuführen und das Hydrogenat aus der genannten Zone der Trennung Flüssigkeit/Gas nach Abtrennung des Katalysators in einer Zone zur Trennung Flüssigkeit/Feststoff abzuziehen, mit Zurückführung des in dem Hydrierungsreaktor dekantierten Katalysators, wobei die genannte Trennung Gas/Flüssigkeit/Feststoff in einer Zone realisiert wird, wo der Transfer Gas-Flüssigkeit begrenzt oder null ist, und mit einer Aufenthaltszeit des dekantierten Katalysators in der genannten Zone der Trennung Gas/Flüssigkeit/Feststoff von unter 30 Minuten.

2. Verfahren nach Anspruch 1, **dadurch gekennzeichnet, daß** die Trennung Gas/Flüssigkeit in einer Anlage durchgeführt wird, gebildet aus einem vertikalen Zylinder, der mindestens eine Speisung aus dem Hydrierungsreaktor für das Reaktionsmedium der Hydrierung, angeordnet in tangentialer Weise an dem vertikalen Zylinder, und mindestens eine Rückführung für das entgaste Reaktionsmedium in den Hydrierungsreaktor umfaßt, angeordnet in tangentialer Weise an dem vertikalen Zylinder in einer niedrigeren Position als die der genannten Zuleitung.

3. Verfahren nach Anspruch 1 oder 2, **dadurch gekennzeichnet, daß** die Trennung Flüssigkeit/Feststoff durch Dekantieren oder Filtration realisiert wird.

4. Verfahren nach Anspruch 3, **dadurch gekennzeichnet, daß** die nach dem Dekantieren oder der Filtration gewonnene Katalysatorsuspension in die Reaktionszone zurückgeführt wird.

5. Verfahren nach einem der Ansprüche 1 bis 4, **dadurch gekennzeichnet, daß** ein Teil des ausgehend von der Zone der Trennung Feststoff/Flüssigkeit in den Hydrierungsreaktor zurückgeführten Katalysators durch neuen Katalysator ersetzt wird.

6. Verfahren nach einem der vorstehenden Ansprüche, **dadurch gekennzeichnet, daß** die Aufenthaltszeit des dekantierten Katalysators in der Zone der Trennung Flüssigkeit/Feststoff unter 15 Minuten beträgt.

7. Verfahren nach Anspruch 6, **dadurch gekennzeichnet, daß** die genannte Aufenthaltszeit unter 5 Minuten beträgt.

8. Verfahren nach einem der vorstehenden Ansprüche, **dadurch gekennzeichnet, daß** das aus der Zone der Trennung Flüssigkeit/Gas abgetrennte Gas in den Hydrierungsreaktor zurückgeführt wird.

9. Verfahren nach einem der vorstehenden Ansprüche, **dadurch gekennzeichnet, daß** der Hydrierungsreaktor ein oder mehrere Rohre in U-Form umfaßt, bei denen die Schenkel vertikal oder leicht geneigt in bezug auf die Vertikale sind, wobei einer der Schenkel von jedem U das Aufsteigen der Dispersion Gas/Flüssigkeit/fester Katalysator zu der Zone der Trennung Flüssigkeit/Gas gewährleistet und der andere Schenkel von jedem U das mindestens teilweise entgaste flüssige Medium aufnimmt, das die Zone der Trennung Flüssigkeit/Gas verläßt.

10. Verfahren nach einem der Ansprüche 1 bis 9, **dadurch gekennzeichnet, daß** die mittlere Konzentration an Katalysator, die dem Gewichtsverhältnis Katalysator/flüssige Reaktionsmischung entspricht, höher als 5 % und vorzugsweise gleich oder höher als 10 % beträgt.

11. Verfahren nach einem der Ansprüche 1 bis 10, **dadurch gekennzeichnet, daß** das eingesetzte aliphatische Dinitril unter den Dinitrilen der allgemeinen Formel (I)
NC-R-CN (I)
ausgewählt wird, in der R eine lineare oder verzweigte Gruppe Alkylen oder Alkenylen mit 1 bis 12 Kohlenstoffatomen darstellt.

12. Verfahren nach einem der Ansprüche 1 bis 11, **dadurch gekennzeichnet, daß** das eingesetzte aliphatische Dinitril unter Adiponitril, Methylglutaronitril, Ethylsuccinonitril, Malononitril, Succinonitril und Glutaronitril sowie ihren Mischungen ausgewählt wird, und insbesondere unter den Mischungen Adiponitril und/oder Methylglutaronitril und/oder Ethylsuccinonitril, die geeignet sind, von einem gleichen Verfahren zur Synthese von Adiponitril zu stammen.

13. Verfahren nach einem der Ansprüche 1 bis 12, **dadurch gekennzeichnet, daß** das eingesetzte aliphatische Dinitril Adiponitril ist.

14. Verfahren nach einem der Ansprüche 1 bis 13, **dadurch gekennzeichnet, daß** der verwendete Katalysator aus einem Katalysator auf der Basis von Raney-Nickel und/oder Raney-Cobalt gebildet wird, der gegebenenfalls, jedoch vorzugsweise ein Dotierungselement umfaßt, ausgewählt unter den Elementen der Gruppen Ib, IVb, VIb, VIIb und VIII des Periodensystem der Elemente, wie es in Handbook of Chemistry and Physics, 51st Edition (1970-1971) veröffentlicht ist.

15. Verfahren nach Anspruch 14, **dadurch gekennzeichnet, daß** der Katalysator auf der Basis von Raney-Nickel und/oder Raney-Cobalt außer Nickel oder Cobalt ein oder mehrere andere Dotierungselemente umfaßt, ausgewählt unter Chrom, Titan, Molybdän, Wolfram, Eisen, Zink, Kupfer.

16. Verfahren nach einem der Ansprüche 1 bis 13, **dadurch gekennzeichnet, daß** der verwendete Katalysator aus einem Metall der Gruppe VIII des Periodensystem der Elemente gebildet wird, wie Ruthenium, Rhodium, Nickel oder Cobalt, abgelagert auf einem Träger, der aus einem Oxid besteht, wie die Aluminiumoxide, die Siliciumoxide, die Aluminosilicate, Titandioxid, Zirkoniumoxid, Magnesiumoxid.

## Claims

1. Process for the continuous hydrogenation of dinitrile to produce at least one corresponding aminonitrile, in the presence of a hydrogenation catalyst which is not dissolved in the reaction medium, **characterized in that** it consists, on leaving the hydrogenation reactor, in carrying out a separation of the gas contained in the reaction medium, in redirecting the gas-depleted reaction medium into the said hydrogenation reactor and in removing the hydrogenate from the said liquid-gas separation zone after separation of the catalyst in a liquid-solid separation zone, with recycling of the decanted catalyst into the hydrogenation reactor, the said gas-liquid-solid separation being carried out in a zone in which the gas-liquid transfer is limited or zero and with a residence time of less than 30 minutes of the decanted catalyst in the said gas-liquid-solid separation zone.

2. Process according to Claim 1, **characterized in that** the gas-liquid separation is carried out in apparatus formed by a vertical cylinder comprising at least one supply of hydrogenation reaction medium from the hydrogenation reactor, arranged tangentially on the vertical cylinder, and at least one recycling of the degassed reaction medium into the hydrogenation reactor, arranged tangentially on the vertical cylinder at a position below that of the said inlet.

3. Process according to Claim 1 or 2, **characterized in that** the liquid-solid separation is carried out by decantation or filtration.

4. Process according to Claim 3, **characterized in that** the suspension of catalyst recovered after decantation or filtration is recycled into the reaction zone.

5. Process according to one of Claims 1 to 4, **characterized in that** some of the catalyst recycled from the solid-liquid separation zone into the hydrogenation reactor is replaced with fresh catalyst.

6. Process according to one of the preceding claims, **characterized in that** the residence time of the decanted catalyst in the liquid-solid separation zone is less than 15 minutes.

7. Process according to Claim 6, **characterized in that** the said residence time is less than 5 minutes.

8. Process according to one of the preceding claims, **characterized in that** the gas separated from the liquid-gas separation zone is recycled into the hydrogenation reactor.

9. Process according to one of the preceding claims, **characterized in that** the hydrogenation reactor comprises one or more U-shaped tubes whose branches are vertical or slightly inclined relative to the vertical, one of the branches of each U allowing for the rise of the gas/liquid/solid catalyst dispersion towards the liquid-gas separation zone, the other branch of each U receiving the at least partially degassed liquid medium leaving the liquid-gas separation zone.

10. Process according to one of Claims 1 to 9, **characterized in that** the average catalyst concentration, corresponding to the catalyst/liquid reaction mixture weight ratio, is greater than 5% and preferably greater than or equal to 10%.

11. Process according to one of Claims 1 to 10, **characterized in that** the aliphatic dinitrile used is chosen from the dinitriles of general formula (I) :
NC-R-CN (I)
in which R represents a linear or branched alkylene or alkenylene group containing from 1 to 12 carbon atoms.

12. Process according to one of Claims 1 to 11, **characterized in that** the aliphatic dinitrile used is chosen from adiponitrile, methylglutaronitrile, ethylsuccinonitrile, malononitrile, succinonitrile and glutaronitrile and mixtures thereof, in particular mixtures of adiponitrile and/or of methylglutaronitrile and/or of ethylsuccinonitrile which can be obtained from the same process for the synthesis of adiponitrile.

13. Process according to one of Claims 1 to 12, **characterized in that** the aliphatic dinitrile used is adiponitrile.

14. Process according to one of Claims 1 to 13, **characterized in that** the catalyst used consists of a catalyst based on Raney nickel and/or on Raney cobalt, optionally, but preferably, including a doping element chosen from the elements from groups Ib, IVb, VIb, VIIb and VIII of the Periodic Table of the Elements as published in the Handbook of Chemistry and Physics, 51st Edition (1970-1971).

15. Process according to Claim 14, **characterized in that** the catalyst based on Raney nickel and/or on Raney cobalt includes, besides nickel or cobalt, one or more other doping elements chosen from chromium, titanium, molybdenum, tungsten, iron, zinc and copper.

16. Process according to one of Claims 1 to 13, **characterized in that** the catalyst used consists of a metal from group VIII of the Periodic Table of the Elements such as ruthenium, rhodium, nickel or cobalt, deposited on a support which is an oxide, such as aluminas, silicas, aluminosilicates, titanium dioxide, zirconium oxide or magnesium oxide.
